(19) Europäisches Patentamt
European Patent Office
Office européen des brevets

(11) Publication number: 0 458 197 A2

# (12) EUROPEAN PATENT APPLICATION

(21) Application number: 91107980.4

(22) Date of filing: 16.05.91

(51) Int. Cl.5: C08F 2/50, C07C 239/06, G03F 7/029

(30) Priority: 17.05.90 IT 2035090

(43) Date of publication of application: 27.11.91 Bulletin 91/48

(84) Designated Contracting States: AT BE CH DE DK ES FR GB LI NL SE

(71) Applicant: MINISTERO DELL' UNIVERSITA' E DELLA RICERCA SCIENTIFICA E TECNOLOGICA
76, Lungotevere Thaon di Revel
I-00196 Roma(IT)

(72) Inventor: Gila, Liliana, Dr. Chem.
4, Viale Fratelli Brignone
I-13039 Trino Vercellese, Vercelli(IT)
Inventor: Ciocca, Paolo, Dr. Chem.
73, Via Pierlombardo
I-28060 Lumellogno, Novara(IT)
Inventor: Morini, Giampiero, Dr. Chem.
215, Via Emilia
I-27058 Voghera, Pavia(IT)
Inventor: Citterio, Attilio, Dr. Chem.
5, Piazza Piola
I-20132 Milano(IT)

(74) Representative: Weinhold, Peter, Dr. et al
Patentanwälte Dipl.-Ing. G. Dannenberg Dr. P. Weinhold Dr. D. Gudel Dipl.-Ing. S. Schubert Dr. P. Barz Siegfriedstrasse 8
W-8000 München 40(DE)

## (54) Use of N-haloamides as photoinitiators for free-radical polymerizations.

(57) Disclosed are N-haloamide photoinitiator compositions for free-radical polymerizations, comprising at least one compound of general formula (I):

R - CO - NHX (I) wherein

R represents an optionally substituted alkyl, cycloalkyl, aryl, alkyl-aryl or aryl-alkyl or heterocyclic group and

X is Cl, Br or I.

EP 0 458 197 A2

The present invention relates to a class of organic compounds which can be used as photoinitiators for free-radical polymerizations.

In particular, the present invention relates to the use of compounds belonging to the class of N-haloamide derivatives as photoinitiator agents in free-radical polymerization reactions.

The present invention furthermore provides new N-haloamides.

As is known, photoinitiators are sources of free radicals which can be activated photochemically and are used as initiators in the polymerization of polymerizable, particularly ethylenically unsaturated substrates. The photoinitiated free-radical polymerizations are of great importance at the industrial level. Photopolymerizable compositions are used, e.g., in the production of printing inks, paints, coatings, photoresists, adhesives, etc.

In general the products available as photoinitiators are compounds such as aryl-ketones, peresters, benzoin ethers, acetophenones, highly-halogenated aryl hydrocarbons, etc.; the latter have recently been abandoned because of their high toxicity.

To find use at an industrial level, a photoinitiator system should be thermally stable and simultaneously labile when it is irradiated with visible and/or U.V. light. Furthermore, to be suitable for a wide range of applications, the system should be active also when irradiated at different wavelengths.

There has now been found a new class of N-haloamide photoinitiators which show the above characteristics.

Therefore, one object of the present invention is to provide a new class of free-radical photoinitiators which are endowed with the requisites of thermal stability and effective photo-decomposability, also at different wavelengths, which renders them particularly suitable for use as photoinitiator agents in polymerization reactions of unsaturated, polymerizable monomers.

Another object is to provide a new class of photoinitiator compounds, which allows the photodecomposition of said compounds to be controlled and regulated by a suitable selection of the chromogenic portions thereof.

A further object is to provide compositions comprising at least one ethylenically unsaturated, polymerizable substrate and at least one of the photoinitiator agents used according to the present invention, as well as the corresponding polymerization process by exposure thereof to visible light or U.V. radiation.

Still another object is the provision of the corresponding polymers derived from monomers polymerized by means of the photoinitiators used according to the present invention.

These and still other objects, which will become clearer for those skilled in the art from the following disclosure, are achieved, according to the present invention, by the use of N-haloamide free-radical photoinitiators of general formula (I):

R - CO - NHX (I)

wherein:

R represents an optionally substituted linear or branched (preferably $C_1$-$C_{20}$) alkyl, (preferably $C_3$-$C_{12}$) cycloalkyl, (preferably $C_6$-$C_{14}$) aryl, (preferably $C_7$-$C_{20}$) alkyl-aryl, (preferably $C_7$-$C_{20}$) aryl-alkyl or a (preferably $C_2$-$C_6$) heterocyclic group;
and

X represents chlorine, bromine or iodine, particularly Cl or Br.

Substituent groups suitable for the R groups are preferably selected from $C_1$-$C_5$ alkyl and alkoxy groups, $NO_2$, halogen (F, Cl, Br and I, particularly F and Cl), and groups of formula

R'-CO-,

in which R', which may be equal to or different from R, has the meanings given above for R.

Also the R'-CO- group can have substituents, preferably selected from $C_1$-$C_5$ alkyl and alkoxy groups, $NO_2$, and halogen.

If more than one substituent is present, said substituents can be equal to or different from each other.

Most preferably, the linear or branched alkyl groups R contain from 1 to 10 carbon atoms, such as methyl, ethyl, propyl, butyl, pentyl and hexyl.

Particularly preferred cycloalkyl groups R are those having 3 to 10 carbon atoms, e.g., cyclopropyl, cyclobutyl, cyclopentyl, cyclohexyl, cycloheptyl and cyclooctyl.

Preferred examples or aryl groups R are phenyl, phenanthryl and anthracyl.

Preferred examples of aralkyl groups R are benzyl and naphthyl(m)ethyl; and preferred alkylaryl groups

comprise, e.g., tolyl, xylyl and cumyl.

Finally, the preferred heterocyclic groups are 5- to 7-membered rings containing 2 to 6, particularly 4 or 5 carbon atoms and one or more (e.g. 2 or 3) heteroatoms selected from 0, N and S, such as morpholyl, piperidyl, thienyl and furanyl.

The preferred R'CO- group, especially when R is phenyl, is the benzoyl group, optionally substituted with the substituents mentioned hereinabove, such as the 2,4,6-trimethyl-or -trimethoxybenzoyl group.

The latter N-halo-benzoyl-benzamide derivatives (R = phenyl and R'CO- = benzoyl) are novel compounds and fall within the scope of the instant invention as such. Preferably, the R'CO- group, when present, is in para-position relative to the -CONHX group.

Particularly advantageous results may be obtained by using the following N-haloamides of general formula (I): N-chloroacetamide, N-bromoacetamide, N-chlorobenzamide, N-chloro-p-bensoyl-benzamide, N-chloro-m-benzoyl-benzamide, N-bromobenzamide, N-bromo-p-benzoyl-benzamide, N-chlorosuccinimide, N-bromosuccinimide, N-bromo-4-methyl-benzamide, N-bromo-4-methoxybenzamide, N-bromotrichloroacetamide, N-bromo-trifluoroacetamide, N-bromo-3-chlorobenzamide, N-chloro-chloroacetamide, N-chloro-di- or -trichloroacetamide, N-chloro-4-nitrobenzamide, N-chlorophenylacetamide, and mixtures thereof.

The N-haloamide photoinitiator compounds according to the present invention are known compounds or can be synthetized according to methods known from literature.

For example, they can be prepared, starting from the corresponding primary or secondary amines, by halogenation with an alkali-metal hypohalogenite [C. Bachand, H. Driguez et al. J.O.C. 39 3136-3138 (1974) ; T. Imamoto et al. Bull. Chem. Soc. of Japan 44 1632-38 (1971)].

Beside the above methods, a tert -butyl hypohalogenite can be used as halogenating agent [J.O.C. 27 4532-4534 (1962)].

As mentioned, the present invention also relates to the novel N-halo-benzoyl-benzamide compounds, i.e., those compounds of general formula (I) in which R = phenyl and R'CO-= benzoyl, i.e., compounds of general formula (II):

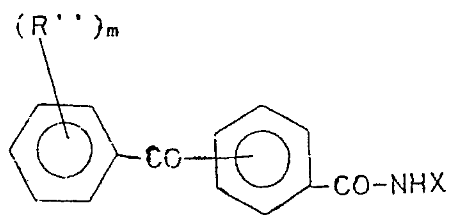

(II)

wherein:

- X is Cl, Br or I;
- R'' represents a $C_1$-$C_5$ alkyl or alkoxy group, $NO_2$ or halogen (F, Cl, Br or I, particularly F, Cl and Br); and
- m is 0 or an integer or from 1 to 3, preferably 0 or 1.

The latter compounds may be obtained, e.g., by reacting, under reflux conditions, a correspondingly substituted benzoyl-benzoic acid with an excess of thionyl chloride in order to obtain benzoyl-benzoyl chloride, from whose acetonic solution the corresponding benzoyl-benzamide may be obtained by treatment with an excess of ammonium acetate and subsequent crystallization. From said benzoyl-benzamide, the desired N-halobenzoyl-benzamide of formula (II) can be obtained by reaction with an alkali-metal hypohalogenite at about 0° C and subsequent neutralization with a mineral or organic acid.

The compounds of formulae (I) and (II) are particularly useful as photoinitiator agents for the polymerization of ethylenically unsaturated, polymerizable substrates, e.g., ethylenically unsaturated monomers and/or prepolymers.

The N-haloamides of formula (I) are usually used as photoinitiators according to molar ratios within the range of from 1:100 to 1:100,000, and preferably of from 1:200 to 1:1,000, relative to the polymerizable monomer or prepolymer present. Mixtures of free-radical photoinitiators of formula (I) can also be used.

The polymerization can be accomplished by exposing the compositions to be polymerized (e.g., the monomer and the photoinitiator) to visible or ultra-violet light, at a suitable wavelength. The wavelength is preferably selected in a way such that it can be absorbed by the chromogenic portion of the photoinitiator compound of formula (I).

In this regard, it is to be noted that the photoinitiators used according to the present invention can be

adapted, by suitably selecting the substituents, to the particular requirements of absorption of radiative energy, relative to the available sources of radiation, the type of substrate, etc.

According to methods known in the art, a light source is usually selected which emits radiation within the range of visible and U.V. light. Therefore, the chromogenic group is preferably selected in a way such that it will absorb light of a wavelength of from 200 to 700 nm.

Examples of photopolymerizable materials comprise monomers or prepolymers containing one or more ethylenic unsaturations, such as the esters of unsaturated monocarboxylic and dicarboxylic acids, e.g., the esters of acrylic, methacrylic, sorbic, fumaric, maleic and itaconic acids with mono- and poly-hydroxy aliphatic, cycloaliphatic and aromatic alcohols containing from 3 to 20 carbon atoms; the amides, N-alkylamides and N-alkoxyamides of acrylic and methacrylic acids; the vinyl esters of $C_2$-$C_{20}$ mono- and di-carboxylic acids; the vinyl ethers of $C_3$-$C_{20}$ monohydroxy and dihydroxy alcohols; the allyl esters of mono- and di-carboxylic acids; the allyl ethers of mono- and di-hydroxy alcohols; vinyl-and vinylidene halo-derivatives; vinylic and styrenic aromatic compounds, etc.; unsaturated polyurethanes; unsaturated polyesters; etc.

The initiator agents of formula (I) according to the present invention are preferably used in the polymerization of methyl methacrylate, methyl acrylate, 2-ethylhexyl acrylate, hydroxyethyl acrylate and methacrylate, ethylene glycol diacrylate, neopentyl glycol diacrylate and dimethacrylate, pentaerythritol triacrylate and tetraacrylate; N,N'-dimethylacrylamide, N-methoxymethacrylamide, N-butoxymethylmethacrylamide; vinyl acetate; vinyl propionate; isobutyl-vinyl-ether, hexyl-vinyl-ether, ethylene glycol divinyl ether, hexanediol divinyl-ether; diallyl maleate, diallyl fumarate; pentaerythritol diallylether; styrene, divinylbenzene and methylstyrene.

The polymerization usually requires an exposure to light of from about 30 seconds to about 20 hours, said time varying as a function of the wavelength, the intensity of the radiation used, the sensitivity of the chromogenic group present, and the unsaturated monomer. The polymerization temperatures are usually within the range of from about 10°C to about 80°C, and preferably of from about 20°C up to about 30°C.

The polymerization reactions initiated with the photoinitiators used according to the present invention may be carried out according to methods known from the literature [J. Polym. Sci.: Polym. Chem. 21 3129-3144 (1983)].

In the following, some examples are given for merely illustrative, non-limitative purposes.

## Example 1

### Synthesis of N-bromobenzamide

A solution of sodium hypobromite is prepared by adding 8 g (0.05 mol) of bromine to 5 g (0.125 mol) of NaOH dissolved in 100 ml of water. To the resulting solution, cooled in an ice bath and kept vigorously stirred, 3 g (0.025 mol) of benzamide are added.

Approximately 7 to 8 minutes later, the reaction mixture - which has turned from yellow into orange-coloured - is rapidly filtered and the filtrate is collected in an Erlenmeyer flask containing 20 ml of an aqueous solution at 30% by volume of acetic acid. The precipitated solid is filtered off, washed with an aqueous solution at 5% by volume of acetic acid and then with water, and finally is dried under vacuum at room temperature.

The crude product is purified by crystallization from dichloroethane; 2.7 g of pure product are thus obtained (purity assayed by iodometry: 99.9%).

According to the same procedure the following compounds are prepared: N-bromo-4-methylbenzamide, N-bromo-4-methoxybenzamide, N-bromo-acetamide, N-bromo-trichloroacetamide, N-bromo-trifluoroacetamide, N-bromo-1-chloropropionamide, N-bromo-3-chlorobenzamide, N-bromo-phenylpropionamide, N-bromo-valeramide, N-bromo-cyclopentamide.

## Example 2

### Synthesis of N-chloroacetamide

2 g (0.034 mol) of acetamide placed in a flask cooled with an ice bath are mixed with 30.4 ml of an aqueous solution of sodium hypochlorite (7% of active chlorine, alkalinity 1.8%). The resulting reaction mixture is kept under vigorous stirring for 15 minutes.

Then 40 to 50 ml of methylene chloride are added and subsequently the reaction mixture is neutralized with about 24 ml of 2N $H_2SO_4$. The organic phase is separated and the aqueous phase is subjected to

continuous extraction with methylene chloride, for 6 to 8 hours. The organic extracts are combined, dried over anhydrous sodium sulfate and are concentrated on a rotary evaporator.

The crude reaction product is purified by crystallization from $CH_2Cl_2$/hexane.

Thus 2.2 g of pure product are obtained (iodometric purity: 98.8%).

According to the same procedure, the following compounds are prepared:
N-chloro-chloroacetamide, N-chloro-dichloroacetamide, N-chloro-trichloroacetamide, N-ohloro-propionamide, N-chloro-2,2-dimethyl-propionamide, N-chloro-hexanamide, N-chloro-cyclohexanamide, N-chloro-benzamide, N-chloro-4-nitrobenzamide, N-chloro-phenylacetamide.

Example 3

Synthesis of N-chloro-4-benzoyl-benzamide

(a) Synthesis of 4-benzoyl-benzoyl chloride

12.7 ml (0.175 mol) of thionyl chloride and a few drops of pyridine are added to 8 g (0.035 mol) of 4-benzoyl-benzoic acid. The reaction mixture is heated to reflux temperature (about 80° C) and is kept at said temperature until the reaction is complete (i.e., until the evolution of hydrogen chloride has ceased).

Thus 8 g of acyl chloride are obtained. The crude reaction product is purified by crystallization from 1:1 ether/pentane.

(b) Synthesis of 4-benzoyl-benzamide

7 g (0.1 mol) of ammonium acetate and 100 ml of acetone are added to 7 g (0.029 mol) of 4-benzoyl-benzoyl chloride.

The reaction mixture is kept under vigorous stirring for one hour at room temperature. The resulting solid is filtered off and the filtrate is concentrated on a rotary evaporator. The crude reaction product is purified by crystallization from toluene.

Thus 3.6 g of pure product are obtained. When the product is submitted to TLC - thin layer chromatography - only one spot is detectable under U.V. light.

(c) Synthesis of N-chloro-4-benzoyl-benzamide

13.9 ml of an aqueous solution of sodium hypochlorite (7% of active chlorine - 1.8% alkalinity) are added to 3.5 g (0.0155 mol) of 4-benzoyl-benzamide in an ice-cooled flask and the resulting reaction mixture is diluted with water to a volume of 30 ml. The reaction mixture is kept under strong stirring for 15 to 20 minutes. Thereafter 10 to 15 ml of methylene chloride are added and, still under strong stirring, 22.4 ml of a 1 N solution of $H_2SO_4$ are added dropwise, until neutrality, over about 15 minutes. The organic phase is separated and the aqueous phase is extracted twice with methylene chloride. The extracts are combined, dried over sodium sulfate and are concentrated on a rotary evaporator.

The crude reaction product is purified by flash chromatography, using 1:1 hexane/acetate as eluent.

Thus 1.5 g of pure product are obtained (iodometric purity: 97.4%). N-chloro-benzoyl-benzamide is a white solid having a melting point of 148° C.

IR (KBr): $\nu$ ketonic CO = $\nu$ amidic CO = 1,600-1,610 $cm^{-1}$. $^1H$-NMR ($CDCl_3$): 8.5 ppm (-NHCl).

Example 4

Synthesis of N-chloro-3-benzoylbenzamide

The process of example 3 is used to prepare N-chloro-3-benzoyl-benzamide.

3-Benzoyl-benzoic acid is treated with thionyl chloride and then with ammonium acetate in acetone to yield 3-benzoyl-benzamide.

Starting from 1 g (4.4 mmol) of 3-benzoylbenzamide treated with 4.4 ml of an aqueous solution of sodium hypochlorite (7% of active chlorine - 1.8% alkalinity) and then neutralized with about 7 ml of 1 N $H_2SO_4$, 200 mg of pure product are obtained (iodometric purity: 97.3%).

The product is a white solid, melting point (m.p.) 128° C.

IR (KBr): $\nu$ ketonic CO = $\nu$ amidic CO = 1,600-1,610 $cm^{-1}$ $^1H$-NMR ($CDCl_3$): 8.5 ppm (-NHCl).

Example 5

Synthesis of N-bromo-4-benzoylbenzamide

2 g (8.8 mmol) of 4-benzoylbenzamide, prepared according to the procedure of example 3b, are added

to a solution of sodium hypobromite, freshly prepared from 44 ml of 1 M NaOH and 2.8 g of bromine and cooled in an ice bath.

The reaction mixture is kept under vigorous stirring for 15 minutes and is then filtered. The filtrate is taken up in 10 ml of an aqueous solution at 30% by volume of acetic acid.

The solid precipitate is filtered off, washed with an aqueous solution at 5% by volume of acetic acid and then with water, and finally is dried under vacuum.

The crude reaction product is crystallized from benzene. Thus 1.1 g of an impure product, still containing the amide used as starting material (iodometric purity: 56.7%), are obtained.

Examples 6 to 16

Photopolymerization of methyl methacrylate (MMA)

(a) Preparation of sample

Commerical methyl methacrylate (MMA) is washed 3 times with a 2% aqueous solution of NaOH (to remove the stabilizer) and then is washed 3 times with distilled water, dried over anhydrous $CaCl_2$ and vacuum-distilled before use.

Exactly 8 ml of monomer are poured into a Pyrex glass (or quartz) test tube equipped with a stopcock and then are deaerated under vacuum by the "freeze/thaw" method. The freeze/thaw cycle is repeated 3 times.

Then an exactly weighed amount of photoinitiator - such as to obtain a solution with a known concentration - is added to the frozen monomer in a nitrogen atmosphere. The solution is stored under nitrogen and is kept protected from light until subjected to irradiation.

(b) Photopolymerization

The photopolymerizations are carried out in a photo-reactor having a ring-like shape (APQ 40 Model, Applied Photophysics Ltd.) by charging a set of samples (maximum 8 samples) into a suitable sample-holder revolving around the UV lamp, immersing it into a water bath and irradiating the solutions for a preset time, at 20°C. At the end of the irradiation period, the test tubes are opened and their contents are poured into an excess of methanol (10:1 volume/volume).

The precipitated polymer is filtered off, washed with methanol, dried under vacuum and weighed. The polymer is then characterized by GPC (gel permeation chromatography).

The photoinitiators used, their initiator/monomer molar ratios, the irradiation times and wavelengths (λ), the conversions and molecular weights obtained are reported in the following Table 1.

Table 1

| Example No. | Initiator | Initiator/monomer molar ratio | Irradiation time (hours) | Irradiation wavelength λ (nm) | Conversion (%) | Mn | Mw | Mw/Mn |
|---|---|---|---|---|---|---|---|---|
| 6 | N-chloro-acetamide | 1/900 | 0.5 | 366 | 2.3 | 192400 | 523700 | 2.7 |
| 7 | N-chloro-benzamide | 1/900 | 0.5 | 366 | 2.7 | 109300 | 294300 | 2.7 |
| 8 | N-chloro-p-benzoyl-benzamide | 1/900 | 0.5 | 366 | 4.7 | 69300 | 266500 | 3.8 |
| 9 | N-chloro-m-benzoyl-benzamide | 1/900 | 0.5 | 366 | 3.1 | 102800 | 322500 | 3.1 |
| 10 | N-bromo-acetamide | 1/900 | 0.5 | 366 | 6.9 | 38700 | 67700 | 1.7 |
| 11 | N-bromo-benzamide | 1/900 | 0.5 | 366 | 10.2 | 28300 | 59100 | 2.0 |
| 12 | N-bromo-p-benzoyl-benzamide | 1/900 | 0.5 | 366 | 11.1 | 23800 | 47500 | 2.0 |
| 13 | N-bromo-succinimide | 1/900 | 0.5 | 366 | 4.4 | 61300 | 136700 | 2.2 |
| 14 | N-bromo-benzamide | 1/900 | 2 | 254 | 7.7 | 159400 | 379600 | 2.4 |
| 15 | N-bromo-benzamide | 1/9000 | 0.5 | 366 | 4.0 | 75700 | 174100 | 2.3 |
| 16 | N-bromo-benzamide | 1/90000 | 0.5 | 366 | 1.2 | 172700 | 625400 | 3.6 |

## Example 17

### Styrene photopolymerization

Commercial styrene is purified before use, as disclosed in examples 6 to 16.

The preparation of the sample, the photopolymerization equipment and the treatments for polymer precipitation and isolation are identical to those disclosed in examples 6 to 16.

A solution, having a molar ratio of initiator to monomer of 1:900, of N-bromobenzamide in styrene is irradiated for 4 hours at 366 nm.

The conversion of polystyrene is 2%, and the polymer has the following molecular weights:

Mn = 14,100
Mw = 35,700
Mw/Mn = 2.5

(The symbols Mn and Mw are used to indicate the number and weight average molecular weights.)

## Claims

1. Use of a compound of general formula (I):

   R - CO - NHX (I)

   wherein:

   R represents an optionally substituted alkyl, cycloalkyl, aryl, alkyl-aryl, aryl-alkyl or heterocyclic group; and

   X represents chlorine, bromine or iodine;

   as free-radical photoinitiator for photopolymerization reactions.

2. Use according to claim 1, wherein R represents a $C_1$-$C_{20}$ alkyl, $C_3$-$C_{12}$ cycloalkyl, $C_6$-$C_{14}$ aryl, $C_7$-$C_{20}$ alkyl-aryl or aryl-alkyl or a 5- to 7-membered heterocyclic group.

3. Use according to any one of claims 1 and 2, wherein the R group is substituted with at least one group independently selected from $C_1$-$C_5$ alkyl and alkoxy groups, $NO_2$, halogen and groups of formula

   R'-CO-

   in which R' has the meanings given for R and is the same as R or different therefrom.

4. Use according to any one of claims 1 to 3, wherein R is selected from $C_1$-$C_{10}$ alkyl, particularly from methyl, ethyl, propyl, butyl, pentyl and hexyl; $C_3$-$C_{10}$ cycloalkyl, particularly from cyclopropyl, cyclobutyl, cyclopentyl, cyclohexyl, cycloheptyl and cyclooctyl; and from phenyl, phenanthryl, anthracyl, benzyl, naphthyl(m)ethyl, tolyl, xylyl and cumyl.

5. Use according to any one of claims 1 to 3, wherein R is selected from $C_2$-$C_6$ heterocyclic groups containing 0 and/or N and/or S heteroatoms, particularly from morpholyl, piperidyl, thienyl and furanyl.

6. Use according to any one of claims 1 to 5, wherein the compound of general formula (I) is selected from N-chloroacetamide, N-bromoacetamide, N-chlorobenzamide, N-chloro-p-benzoyl-benzamide, N-chloro-m-benzoyl-benzamide, N-bromobenzamide, N-bromo-p-benzoyl-benzamide, N-chlorosuccinimide, N-bromosuccinimide, N-bromo-4-methylbenzamide, N-bromo-4-methoxybenzamide, N-bromotrichloroacetamide, N-bromo-trifluoroacetamide, N-bromo-3-chlorobenzamide, N-chloro-chloroacetamide, N-chloro-di- or trichloroacetamide, N-chloro-4-nitrobenzamide, N-chlorophenylacetamide, and mixtures thereof.

7. Photopolymerizable composition, comprising at least one ethylenically unsaturated, polymerizable substrate and at least one compound of general formula (I) as defined in any one of claims 1 to 6.

8. Composition according to claim 7, in which the compound of general formula (I) and the polymerizable material are present in a molar ratio of from 1:100 to 1:100,000, particularly 1:200 to 1:1000.

9. Composition according to any one of claims 7 and 8, in which the ethylenically unsaturated substrate comprises methyl methacrylate and/or styrene.

**10.** Process for polymerizing a photopolymerizable composition according to any one of claims 7 to 9, comprising exposing said composition to radiation having a wavelength which can be absorbed by the chromogenic group of the photoinitiator, preferably a wavelength of from 200 to 700 nm.

**11.** Process according to claim 10, in which the polymerization is carried out at a temperature of from about 10 to about 80° C, preferably of from about 20 to about 30° C, for intervals of from about 30 seconds to about 20 hours.

**12.** N-Halo-benzoyl-benzamides of general formula (II):

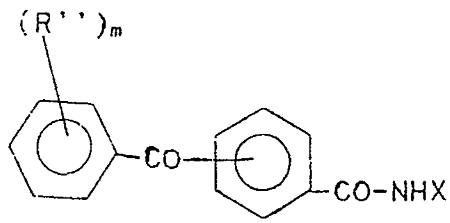

(II)

wherein

X = Cl, Br or I;

R'' represents a $C_1$-$C_5$ alkyl or alkoxy group, $NO_2$ or halogen,

m is 0 or an integer of from 1 to 3.

**13.** Compound according to claim 12, i.e., N-chloro-4-benzoyl-benzamide, N-chloro-3-benzoyl-benzamide and N-bromo-4-benzoyl-benzamide.

**14.** Polymers, obtainable by the process according to any one of claims 10 and 11.